# EUROPÄISCHE PATENTSCHRIFT

(11) **EP 0 379 962 B1**
(45) Veröffentlichungstag und Bekanntmachung des Hinweises auf die Patenterteilung: **11.08.1993**
(21) Anmeldenummer: 90100951.4
(22) Anmeldetag: 18.01.1990
(51) Int. Cl.: A61F 11/00, A61F 2/18

(54) **Einsatzteil zur Belüftung des Mittelohrraumes**
Middle ear ventilation insert
Insert pour ventiler l'oreille moyenne

(30) Priorität: 21.01.1989 DE 3901795
(43) Veröffentlichungstag der Anmeldung: 01.08.1990
(73) Patentinhaber: Kurz, Heinz, D-72144 Dusslingen (DE)
(72) Erfinder: Kurz, Heinz, D-72144 Dusslingen (DE)
(74) Vertreter: Möbus, Rudolf, Dipl.-Ing.

(56) Entgegenhaltungen:
- EP-A- 0 287 430
- GB-A- 2 069 339
- US-A- 3 815 577
- US-A- 4 015 607
- US-A- 4 305 395

## Beschreibung

Die Erfindung betrifft ein Einsatzteil zur Belüftung des Mittelohrraumes nach dem Oberbegriff des Anspruchs 1.

Aus der US-A-4 015 607 ist bereits eine Belüftungskanüle aus Kunststoff- oder Silikonmaterial bekannt, die beim Einsetzen mit einem Draht versteift wird, der nach dem Einsetzen wieder herausgezogen wird. Es hat sich gezeigt, daß bei einem Teil der Patienten diese Kunststoffkanülen Abstoßreaktionen des Körpers und Entzündungen auslösen. Außerdem ist bei der bekannten Kanüle die Gefahr gegeben, daß beim Herausziehen des Versteifungsdrahtes auch die gesamte Kanüle wieder zurückgezogen wird. Dies ist insbesondere bei denjenigen Patienten zu befürchten, bei denen der Verbindungskanal zwischen Mittelohrraum und Nasen-Rachen-Raum starke Krümmungen aufweist.

Der Erfindung liegt die Aufgabe zugrunde, ein Einsatzteil der eingangs genannten Art zu schaffen, das keine Körperabwehrreaktionen hervorruft und dessen Einsatz auch bei schwierigen anatomischen Verhältnissen gewährleistet ist.

Die gestellte Aufgabe wird erfindungsgemäß mit einem Einsatzteil mit den Merkmalen des Hauptanspruchs gelöst.

An dem in der trichterförmigen Erweiterung des Röhrchenendes abgestützten Haltestopfen läßt sich eine Pinzette ansetzen, mit welcher das eingesetzte Feingoldröhrchen beim Herausziehen des Versteifungsdrahtes in seiner Einsatzlage zurückgehalten werden kann. Nach dem Herausziehen des flexiblen Drahtes wird dann auch der Haltestopfen entfernt.

Für Fälle, in denen der Verbindungskanal zwischen Mittelohrraum und Nasen-Rachen-Raum starke Krümmungen aufweist, kann das Einsatzteil auch aus einem Feingolddraht bestehen, der mit mindestens einer über seine ganze Einführungslänge durchgehenden Längsnut als Luftführungskanal versehen ist.

Feingold hat sich als durchweg körperverträglich erwiesen. Der Anwendungszweck rechtfertigt seinen relativ hohen Materialpreis. Die erforderliche Feingoldmenge ist relativ gering, weil sich das Feingoldröhrchen oder der Feingolddraht mit einem sehr kleinen Durchmesser mit ausreichender Stabilität herstellen läßt und auch ein kleiner Durchlaßquerschnitt den Zweck des Einsatzteiles erfüllt.

Nachfolgend werden zwei Ausführungsbeispiele des Erfindungsgegenstandes anhand der beiliegenden Zeichnung näher erläutert.

Im einzelnen zeigen:
- Fig. 1: eine Seitenansicht eines als Röhrchen ausgebildeten Einsatzteiles;
- Fig. 2: eine Seitenansicht des Versteifungsdrahtes des Röhrchens mit einem aufgeschobenen Haltestopfen;
- Fig. 3: einen gegenüber Fig. 1 vergrößerten Längsschnitt durch eines der Enden des Röhrchens;
- Fig. 4: eine Einzeldarstellung des Haltestopfens in gegenüber Fig. 2 vergrößertem Maßstab;
- Fig. 5: eine Seitenansicht eines als Draht ausgebildeten Einsatzteiles;
- Fig. 6: einen Querschnitt durch den Draht entlang der Linie VI - VI in Fig. 5 in gegenüber Fig. 5 vergrößertem Maßstab;
- Fig. 7: eine Darstellung des vorderen Endes des Drahtes in gegenüber Fig. 5 vergrößertem Maßstab;
- Fig. 8: eine Darstellung des hinteren, mit einem Griffkopf versehenen Endes des Drahtes in gegenüber Fig. 5 vergrößertem Maßstab.

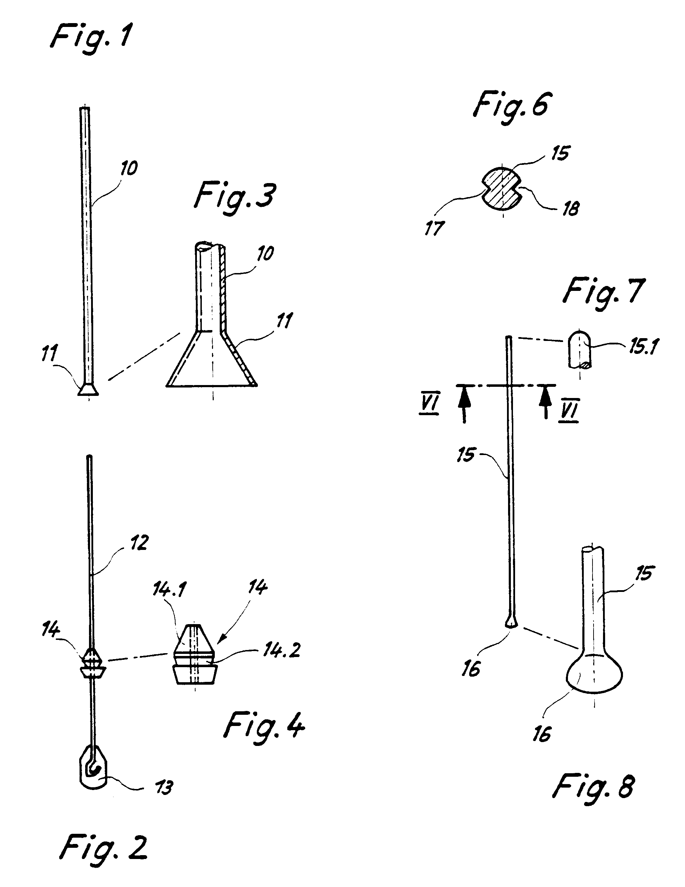

Fig. 1 zeigt ein als Einsatzteil dienendes Feingoldröhrchen 10 etwa in doppelter Größe. Das Feingoldröhrchen 10 weist an seinem in Einsetzrichtung gesehen hinteren Ende eine trichterförmige Erweiterung 11 auf. Vor dem Einsetzen des Feingoldröhrchens 10 wird zu seiner Versteifung ein aus Fig. 2 ersichtlicher flexibler Draht 12 in das trichterförmig erweiterte Ende des Feingoldröhrchens 10 eingeschoben. Der Draht 12 endet auf einer Seite in einer Griffplatte 13. Auf den Draht 12 ist ein Haltestopfen 14 aufgeschoben, der im einzelnen aus Fig. 4 ersichtlich ist. Er weist einen kegelförmigen, in die trichterförmige Erweiterung 11 des Feingoldröhrchens 10 passenden Endabschnitt 14.1 auf. An seinem aus der trichterförmigen Erweiterung 11 des Feingoldröhrchens 10 herausragenden Teil ist er mit einer Ringnut 14.2 versehen, in welcher eine Haltepinzette angesetzt werden kann.

Nach dem Einsetzen des Feingoldröhrchens wird mittels einer am in die trichterförmige Erweiterung 11 ragenden Haltestopfen 14 angesetzten Pinzette das Feingoldröhrchen in seiner Einsatzlage zurückgehalten, während der Versteifungsdraht 12 aus dem Feingoldröhrchen 10 herausgezogen wird. Anschließend wird auch der Haltestopfen 14 entfernt.

Die Fig. 5 bis 8 zeigen als Einsatzteil einen Feingolddraht 15. Er ist - wie auch der Versteifungsdraht 12 des Ausführungsbeispiels nach Fig. 1 - 4 - an seinem vorderen Ende 15.1 sondenartig abgerundet. An seinem anderen Ende ist der Feingolddraht 15 zu einem Haltekopf 16 geformt. Aus der Schnittdarstellung der Fig. 6 sind zwei sich über seine gesamte Einsatzlänge erstreckende Längsnuten 17 und 18 ersichtlich, die auf der Außenseite des Feingolddrahtes 15 ausgebildet sind. Es kann auch nur eine einzige Nut sein. Der Feingolddraht 15 ist für Sonderfälle vorgesehen, wo durch starke Krümmungen und Verengungen des Verbindungskanales zwischen Mittelohrraum und Nasen-Rachen-Raum eines Patienten das Einschieben der Einsatzteile besondere Schwierigkeiten macht.

## Patentansprüche

1. Einsatzteil zur Belüftung des Mittelohrraumes, das aus einem in den Verbindungskanal zwischen Mittelohrraum und Nasen-Rachen-Raum einschiebbaren länglichen Körper kleinen Durchmessers besteht, in welchem mindestens ein Luftführungskanal ausgebildet ist, dadurch gekennzeichnet, daß der Körper ein Feingoldröhrchen (10) ist, dessen in Einsetzrichtung gesehen hinteres Ende (11), aus welchem ein in an sich bekannter Weise zur Versteifung eingeschobener flexibler Draht (12) nach dem Einsetzen des Feingoldröhrchens (10) herausgezogen wird, zur Aufnahme des kegelförmigen Endes (14.1) eines auf dem Versteifungsdraht (12) längsverschiebbar gelagerten Haltestopfens (14) trichterförmig erweitert ist.

2. Einsatzteil nach Anspruch 1, dadurch gekennzeichnet, daß es aus einem Feingolddraht (15) besteht, der mit mindestens einer über seine ganze Einführungslänge durchgehenden Längsnut (17, 18) als Luftführungskanal versehen ist.

3. Einsatzteil nach Anspruch 1 oder 2, dadurch gekennzeichnet, daß sein Außendurchmesser höchstens 1 mm beträgt.

## Claims

1. An insert for ventilating the middle ear cavity which consists of an elongated body of small diameter which can be inserted in the connecting duct between the middle ear cavity and the nasal-pharyngeal cavity, in which elongated body at least one air conduction duct is constructed, characterised in that the body is a small pure gold tube (10), the rear end (11) of which, as viewed in the direction of insertion and from which a flexible wire (12) inserted for reinforcement in the manner known in the art can be withdrawn after inserting the small pure gold tube (10), is conically enlarged in order to receive the conical end (14.1) of a retention plug (14), which is longitudinally displaceable on the reinforcing wire (12) .

2. An insert according to claim 1, characterised in that it consists of a pure gold wire (15) which is provided with at least one longitudinal channel (17, 18) continuing over its entire length of insertion as an air conduction duct.

3. An insert according to claim 1 or 2, characterised in that its outside diameter is 1 mm at most.

## Revendications

1. Insert pour ventiler l'oreille moyenne qui est constitué d'un corps allongé de faible diamètre pouvant être inséré dans le canal de liaison entre l'oreille moyenne et le rhino-pharynx, dans lequel est formé au moins un canal d'amenée d'air, caractérisé en ce que le corps est un petit tube d'or fin (10), dont l'extrémité arrière (11) vue dans le sens de l'insertion, de laquelle est tiré d'une façon connue en soi un fil flexible (12) inséré aux fins de rigidification, après l'insertion du petit tube d'or fin (10), pour réception de l'extrémité conique (14.1) d'un bouchon de maintien (14) monté coulissant longitudinalement sur le fil de rigidification (12), est évasée en forme d'entonnoir.

2. Insert selon la revendication 1, caractérisé en ce qu'il est constitué d'un fil d'or fin (15) qui est muni d'au moins une rainure longitudinale (17, 18) traversant toute sa longueur d'insertion comme canal d'amenée d'air.

3. Insert selon la revendication 1 ou 2, caractérisé en ce que son diamètre extérieur est de 1 mm maximum.
